# EUROPEAN PATENT SPECIFICATION

(11) **EP 1 418 229 B1**
(45) Date of publication and mention of the grant of the patent: **22.04.2009**
(21) Application number: 02738901.4
(22) Date of filing: 02.07.2002
(51) Int. Cl.: C12N 9/16, C12N 9/96

(54) **METHOD OF STABILIZING ALKALINE PHOSPHATASE**
VERFAHREN ZUR STABILISIERUNG VON ALKALISCHER PHOSPHATASE
PROCEDE DE STABILISATION DE LA PHOSPHATASE ALCALINE

(30) Priority: 02.07.2001 JP 2001200612
(43) Date of publication of application: 12.05.2004
(73) Proprietor: Asahi Kasei Pharma Corporation, Tokyo 101-8481 (JP)
(72) Inventor: UEDA, Shigeru, Tagata-gun Shizuoka 410-2124 (JP); HIRAYAMA, Toshiaki, Tagata-gun, Shizuoka 410-2321 (JP)
(74) Representative: Forstmeyer, Dietmar
(86) International application number: PCT/JP2002/006673
(87) International publication number: WO 2003/004633

(56) References cited:
- EP-A2- 0 037 110
- WO-A1-96/03034
- JP-A- 4 330 280
- JP-A- 6 284 885
- JP-A- 8 187 095
- JP-A- 8 228 774
- ARAKAWA TSUTOMU ET AL: "Factors affecting short-term and long-term stabilities of proteins" ADVANCED DRUG DELIVERY REVIEWS, vol. 46, no. 1-3, 1 March 2001 (2001-03-01), pages 307-326, XP002318382 ISSN: 0169-409X
- RESSING M E ET AL: "THE INFLUENCE OF SUCROSE DEXTRAN AND HYDROXYPROPYL-BETA-CYCLODEXTRIN AS LYOPROTECTANTS FOR A FREEZE-DRIED MOUSE IGG2A MONOCLONAL ANTIBODY (MN12)" PHARMACEUTICAL RESEARCH, NEW YORK, NY, US, vol. 9, no. 2, 1992, pages 266-270, XP000881705 ISSN: 0724-8741
- HORA M ET AL: "LYOPHILIZED FORMULATIONS OF RECOMBINANT TUMOR NECROSIS FACTOR" PHARMACEUTICAL RESEARCH, NEW YORK, NY, US, vol. 9, no. 1, 1992, pages 33-36, XP002915357 ISSN: 0724-8741
- IZUTSU K-I ET AL: "THE EFFECTS OF ADDITIVES ON THE STABILITY OF FREEZE-DRIED BETA GALACTOSIDASE STORED AT ELEVATED TEMPERATURE" INTERNATIONAL JOURNAL OF PHARMACEUTICS (KIDLINGTON), vol. 71, no. 1-2, 1991, pages 137-146, XP002318383 ISSN: 0378-5173
- HINRICHS W L J ET AL: "Inulin glasses for the stabilization of therapeutic proteins" INTERNATIONAL JOURNAL OF PHARMACEUTICS (KIDLINGTON), vol. 215, no. 1-2, 14 March 2001 (2001-03-14), pages 163-174, XP002318384 ISSN: 0378-5173
- CHUNG I S ET AL: "Production of human alkaline phosphatase, a secreted, glycosylated protein, from a baculovirus expression system and the attachment-dependent cell line Trichoplusia ni BTI-Tn 5B1-4 using a split-flow, air-lift bioreactor." BIOTECHNOLOGY PROGRESS. 1993 NOV-DEC, vol. 9, no. 6, November 1993 (1993-11), pages 675-678, XP002318385 ISSN: 8756-7938
- FORD A.W. ET AL.: 'The effect of carbohydrate additives in the freeze-drying of alkaline phosphatase' J. PHARM. PHARMACOL. vol. 45, no. 2, 1993, pages 86 - 93, XP002955689
- FORD A.W. ET AL.: 'The adverse effect of glycation of human serum albumin on its preservative activity in the freeze-drying and accelerated degradation of alkaline phosphatase' J. PHARM. PHARMACOL. vol. 45, no. 10, 1993, pages 900 - 906, XP002955690
- NOSJEAN O. ET AL.: 'Human tissue non-specific alkaline phosphatases: sugar-moiety-induced enzymic and antigenic modulations and genetic aspects' BIOCHEM. J. vol. 321, 1997, pages 297 - 303, XP002955691
- HAFKENSCHEID & JANSEN: CLIN. CHIM. ACTA, vol. 59, 1975, pages 63-69,

## Description

### TECHNICAL FIELD

The present invention relates to a freeze-dried alkaline phosphatase-containing preparation for use in clinical examination. Specifically, the present invention relates to a method of stabilizing human liver alkaline phosphatase in a freeze-dried preparation. More specifically, the present invention relates to a freeze-dried preparation which shows no increase in activity after reconstitution in water and can be stored for a prolonged period of time and a method of stabilizing the same.

### BACKGROUND ART

Alkaline phosphatase (ALP) is an enzyme which is known to be existent in plants, animals, microorganisms, or the like. For example, their animal origins are wide-ranging such as the small intestines of bovines, pigs, rabbits, and dogs and the kidneys of bovines and pigs, human placentas, and its microorganisms sources include enzymes derived from E. coli. All of them are available on the market. Those enzymes are the same in function but differ from one another in properties such as specificity, thermal stability, and reactivity.

In a clinical diagnosis, the enzymatic activity in a human serum which reflects a variety of morbid states is measured. The sources of an enzyme contained in the serum are wide-ranging such as the liver, kidney, bone, small intestine, and placenta.

In general, the enzymatic activity is represented by a relative numerical value which changes according to conditions such as pH and measurement temperature. Therefore, even when the same sample is used, the activity value differs according to a reagent used. Factors such as the deterioration of a reagent and the deterioration of a sample itself have a great influence upon the activity value. Therefore, in order to accurately measure the enzymatic activity, a reference material which is stable without variations must be used each time a measurement is made to compare its measurement results with the measurement results of the sample, or a standard measurement method must be used.

In the field of clinical examination, commercially available products under the common name of control serum, calibrator, or reference material are distributed as the reference material for the measurement of the enzymatic activity according to its application. Of those, the control serums are used for internal quality control, that is, daily control at a certain measurement facility, and the calibrators and the reference materials are used not only for quality control but also as a control substance between facilities while taking into account the accuracy of a measurement value.

However, the enzymatic activity of ALP in the human serum easily changes and the change rate of activity in fresh blood after 96 hours is -4% to 10% according to storage conditions. It has been reported that when a frozen pooled serum was dissolved and stored at room temperature, the activity was raised by 6.4% (Clin. Chem., vol. 18(4), 1972). It is known that thermal stability differs according to the type of an isozyme (Akio Genba, Isoenzyme, Igaku-Shoin Ltd., pp. 10-16, 1978).

Meanwhile, freeze-drying is useful and commonly used for the long-term storage of a substance which is unstable to heat and easily denatured when it is left to stand in an aqueous solution form, such as a protein. Enzymes are often freeze-dried to maintain their activities for the long term. But, there are a large number of enzymes which are easily denatured and deactivated in the course of freeze-drying depending on their type and purity. Therefore, a protein such as albumin and a saccharide such as sucrose or trehalose are added as stabilizers to a target substance in order to eliminate this problem. For example, JP-A 56-148291 discloses stabilizers for enzymes which include sucrose and bovine or human serum albumin. However, there is no stabilizer which has an effect on all kinds of proteins, and studies on an effective stabilizer for each protein are now under way.

For quality control and other purposes, various reports on the freeze-drying of ALP have been made. For example, as for the effect of adding a saccharide to ALP, which affects freeze-drying, trehalose, mannitol, and lactose are compared (J. Pharm. Pharmacol., 45(10), pp.86-93, 1993). According to this report, trehalose has a larger stabilization effect than lactose and lactose has a larger stabilization effect than mannitol. The used enzyme is only a roughly purified enzyme derived from the mucous membrane of the bovine small intestine and a human-derived enzyme is not studied. It is described in J. Pharm, Pharmacol., 45(10), pp.900-906, 1993 that when freeze-drying is carried out by using a purified enzyme derived from the mucous membrane of the bovine small intestine and glycated human serum albumin as an additive, a negative effect is given. Since the saccharified albumin is obtained by combining a reduced saccharide with albumin, it is not preferred to use this combination as an additive. It is reported that when 15% of trehalose is used as the only additive, ALP activity is lost in the freeze-drying process and the residual activity of about 40% is only obtained after drying as Comparative Example.

As described above, the reference material must be stable for a warranty period, generally 1 year or more and show a certain performance when in use. Up to now, it has been pointed out that when a commercially available freeze-dried control serum is reconstituted, ALP shows a gradual increase in its activity (Clin. Chem. Vol.18(4), pp.366-377, 1972).

It cannot be said that this is preferred when used after reconstitution. For comparison between quality control substances obtained by freezing the human serum and by freeze-drying the human serum, in the case of ALP and creatine kinase (CK) , it is reported that frozen products are superior as control serums (Clin. Biochem., 29 (2), pp. 183-185, 1996). Those control serums are mostly prepared by adding an animal-derived enzyme to a serum in addition to intrinsic human-derived ALP (Cli. Chem., 35(3), p.510, 1989). Although human-derived enzymes should have been used, in actuality, a variety of animal-derived enzymes which differ from one another in properties have often been used from the viewpoints of ethics, infection, and acquisition ease (Clin. Chem., 33(11), pp.1971-1977, 1987).

In recent years, it has been easy to extract enzymes from established animal cells and recombinant cells obtained by using genetic engineering without using biomaterials, and commercial products including a human-derived enzyme are now available. For example, a control serum containing many freeze-dried enzymes including ALP acquired from a human amnion cell line shows an increase in activity after reconstitution (Analysis of Bio Specimens, 14(2), pp.81-89, 1991: Clinical examination/equipment/reagent, 15(4), pp.615-623, 1992).

The development of quality control substances including the human-derived ALP is being accelerated from now on. However, a freeze-dried product which includes the human-derived ALP, does not show an increase in activity after reconstitution in water, and is stable for the long term storage is not reported yet.

It is an object of the present invention to provide a freeze-dried preparation which includes human liver ALP, does not show an increase in activity after reconstitution in water, and is stable for the long term and a method of stabilizing human liver ALP in the preparation.

### DISCLOSURE OF THE INVENTION

The inventors of the present invention have conducted intensive studies to solve the above problem and have found that a composition which shows small variations in activity before and after freeze-drying and is stable for the long term can be obtained unexpectedly by adding a saccharide selected from the group consisting of galactose, lactose and fructose, and albumin or dextran at the time of freeze-drying human liver ALP. The present invention has been accomplished based on this finding. That is, according to the present invention, there is provided a human liver ALP stabilizing preparation which can be preserved for the long term, is highly stable in practical use, and shows no variations such as an increase in activity when it is used, that is, it is reconstituted, by freeze-drying human liver ALP in the presence of a saccharide selected from the group consisting of galactose, lactose and fructose, and albumin or dextran.

The present invention will be described in detail thereinafter. The saccharide which can be used in the freeze-dried preparation of the present invention is preferably selected from the group consisting of galactose, lactose and fructose, and a concentration of the saccharide is preferably 0.5 to 20 (W/V)%, more preferably 1 to 10 (W/V) %. Those saccharides may be used alone or as a mixture of two or three.

The albumin may be mammalian albumin such as human or bovine serum albumin (BSA), or avian albumin such as chicken serum albumin, and the concentration of the albumin is preferably 0.3 to 7 (W/V)%, more preferably 1 to 5 (W/V)%. Further, what is obtained by culturing a recombinant cell with a gene for encoding amino acid of each albumin and purified may also be used. The albumin is used as an excipient. Other proteins and polysaccharides such as dextran which show the same effect as albumin may be appropriately used alone or in combination. An amount of dextran is preferably 0.3 to 7 (W/V) %, more preferably 1 to 5 (W/V)% when in use.

The alkaline phosphatase which can be used in the present invention is a human liver-derived enzyme. The liver, kidney, bone, small intestine, and placenta are known as the sources of human-derived ALP and an enzyme can be obtained from those biomaterials. The placenta-derived enzyme is available on the market and the acquisition of the enzyme from those biomaterials is not preferred from the viewpoints of ethics and infection. It can be obtained from the cultured products of cell lines such as HeLa cell lines and amnion cell lines. Further, genes for encoding a human-derived ALP protein are known by recent progress made in genetic engineering technology and the enzyme can be obtained from transformed cells including those genes. For example, liver type ALP is acquired from a transformed animal cell, purified and marketed from Asahi Kasei Co., Ltd. (catalog for diagnostic enzymes of Asahi Kasei Co., Ltd.).

Here, the human liver type ALP gene is identical to what is called human organ non-specific ALP gene. Also in this case, as the transformed cell may be used not only human cells but also animal cells other than the human cells such as Chinese hamster-derived CHO cell and microbe cells such as E. coli, yeasts, and molds. A transformed gene for encoding an ALP derivative obtained by deleting or substituting part of the amino acid sequence of ALP and adding other amino acid residue or amino acid sequence may also be used. Those produced enzymes except the commercially available ones may be used for this purpose after their purities are improved to a practical level by combining purification methods of public knowledge such as column chromatography.

An addition amount of ALP in the freeze-dried preparation of the present invention is not particularly limited but preferably 9 to 6500 U/L, more preferably 45 to 1300 U/L.

The pH of the aqueous solution before freeze-drying is around neutral, specifically around 6.5 to 8.5. Since the pH of the solution in which a freeze-dried product is reconstituted is desirably within the same range, a suitable buffer, for example, a Good's buffer such as PIPES, HEPES, or BES, phosphate buffer, or Tris buffer may be used in a concentration of 5 to 200 mM, specifically 10 to 100 mM. Various additives used to improve the form of a freeze-dried preparation, such as dextran, dextran sulfate, and sugar alcohol such as mannitol may be suitably used.

Since it is known that ALP has zinc in its molecule, for example, zinc chloride is added, or magnesium chloride known as an activating agent may be suitably added. Amino acid which is known to have the effect of stabilizing an enzyme, such as valine may be suitably added.

### BEST MODE FOR CARRYING OUT THE INVENTION

The present invention will be described based on Examples.

### (Example 1)

(1) BSA was not added, (2) 1 (W/V)% of BSA was added, and (3) 3 (W/V)% of BSA was added to a 20 mM POPSO-NaOH buffer (pH 7.5) containing 5% of lactose, 0.5 mM of magnesium chloride, 10 µM of zinc chloride and 400 U/L of human liver-derived ALP (Number T-73 produced by Asahi Kasei Co., Ltd.), and 2 ml of each of the obtained solutions was injected into a vial and freeze-dried. Under the above respective conditions, a temperature acceleration test was made on each of the freeze-dried products at 37°C which was then reconstituted in 2 ml of distilled water every week for up to 4 weeks at the time of measurement to measure ALP activity. Changes in the residual activity of each sample are shown in Table 1. Stabilization was significantly observed in BSA-added systems compared with a BSA-free system.

**Table 1**

| Residual activity (%) | | | |
|---|---|---|---|
| | No BSA | 1% of BSA | 3% of BSA |
| 1 week | 91.2 | 92.0 | 91.4 |
| 2 weeks | 84.8 | 86.4 | 86.6 |
| 3 weeks | 76.7 | 82.6 | 84.1 |
| 4 weeks | 66.2 | 80.4 | 82.7 |

### (Example 2)

3% of sucrose, 3% of galactose, and 3% of lactose were each added to a 20 mM BES-NaOH buffer (pH 7.5) containing 3% of BSA, 0.1 mM of zinc chloride, 30 mM of valine, and 400 U/L of human liver-derived ALP, and 3 ml of each of the obtained solutions was injected into two vials and freeze-dried. 3 ml of distilled water was added to each of the freeze-dried products to reconstitute it in order to measure ALP activity. The remaining one vial was placed in an incubator at 37°C and left to stand for one week. Thereafter, the activity of ALP was measured in the same manner. As shown in Table 2, the products added with galactose and lactose of the present invention significantly showed the higher residual activity than that of the system added with sucrose.

**Table 2**

| Residual activity (%) | | | |
|---|---|---|---|
| | 3% of sucrose | 3% of galactose | 3% of lactose |
| 1 week | 75 | 85.2 | 84.7 |

### (Example 3)

5 (W/V)% of galactose, 5 (W/V)% of fructose, and 5 (W/V)% of lactose were each added to a 40 mM PIPES-NaOH buffer containing 3% of BSA, 0.5 mM of magnesium chloride, 10 µM of zinc chloride, and 400 U/L of human liver-derived ALP (Number T-73 produced by Asahi Kasei Co, Ltd.), and 2 ml of each of the obtained solutions was injected into a vial and freeze-dried. As for freeze-drying conditions, after evacuation at a freezing temperature of -50°C, primary drying was carried out at -10°C for 12 hours, secondary drying was carried out at 20°C for 24 hours, and vacuum capping was carried out. 2 ml of distilled water was added to each of the freeze-dried products to dissolve it in order to measure ALP activity of each saccharide-added product. The remaining vials were placed in an incubator at 37°C and dissolved in 2 ml of distilled water one for each week to measure the activity of ALP for up to 3 weeks. The residual activity is shown in Table 3 when the enzymatic activity right after freeze-drying is 100%. The residual activity of ALP when galactose, fructose, and lactose were each added was significantly high compared with that when no saccharide was added.

**Table 3**

| Residual activity (%) | | | | |
|---|---|---|---|---|
| | No saccharide | 5% of galactose | 5% of fructose | 5% of lactose |
| 1 week | 68.5 | 93.9 | 92.4 | 88.9 |
| 2 weeks | 66.2 | 92.4 | 90.1 | 86.1 |
| 3 weeks | 59.3 | 90.7 | 88.6 | 87.1 |

### (Example 4)

(1) Saccharide was not added, (2) 1 (W/V)% of galactose was added, and (3) 3 (W/V)% of galactose was added, (4) 5 (W/V)% of galactose was added, and (5) 3 (W/V)% of mannose was added to a 40 mM BES-NaOH buffer containing 3% of BSA, 0.5 mM of magnesium chloride, 10 µM of zinc chloride, and 400 U/L of human liver-derived ALP (Number T-73 produced by Asahi Kasei Co, Ltd.), and 2 ml of each of the obtained solutions was injected into a vial and freeze-dried. Under the above respective conditions, a temperature acceleration test was made on each of the freeze-dried products at 37°C which was then dissolved in 2 ml of distilled water every week for up to 4 weeks at the time of measurement to measure ALP activity. Changes in the residual activity of each sample are shown in Table 4. As confirmed from the table, although the addition of galactose yielded an effect, the product added with mannose showed an abrupt drop in activity after 3 weeks.

**Table 4**

| Residual activity (%) | | | | | |
|---|---|---|---|---|---|
| | No saccharide | 1% of galactose | 3% of galactose | 5% of galactose | 3% of mannose |
| 1 week | 81.8 | 88.3 | 94.9 | 97.0 | 95.0 |
| 2 weeks | 79.6 | 86.7 | 93.0 | 98.6 | 88.3 |
| 3 weeks | 75.9 | 83.3 | 89.5 | 92.7 | 69.8 |
| 4 weeks | 75.1 | 81.5 | 88.3 | 89.9 | 48.4 |

### (Example 5)

5 (W/V)% of galactose or 5 (W/V)% of 3-lactose was added to a 40 mM BES-NaOH buffer containing 1% of BSA, 3% of dextran 60K, 0.5 mM of magnesium chloride, 10 µM of zinc chloride, and 400 U/L of human liver-derived ALP (Number T-73 produced by Asahi Kasei Co., Ltd.), and 2 ml of each of the obtained solutions was injected into a vial and freeze-dried. After the freeze-dried products were dissolved and their initial activities were measured, every four vials were placed in incubators at 25°C, 37°C and 45°C to carry out an accelerated degradation test. The results are shown in Table 5. Then, based on the results, the period of time of each sample until its residual activity became 98% when it was preserved at 4°C and -10°C was calculated using the Arrhenius expression (J. Biol. Stand., 12, pp.195-224, 1984) and shown in Table 6. The calculation results show that the galactose solution and the lactose solution would be stable for 1 or more years when they were kept at 4°C and for about 15 years when they were kept at -10°C.

**Table 5**

| Residual activity (%) | | | | | | |
|---|---|---|---|---|---|---|
| | 5% of galactose | | | 5% of lactose | | |
| | load at 25°C | load at 37°C | load at 45°C | load at 25°C | load at 37°C | load at 45°C |
| 1 week | 99.8 | 98.0 | 94.3 | 98.9 | 96.1 | 92.0 |
| 2 weeks | 99.3 | 96.1 | 73.2 | 96.9 | 94.3 | 70.6 |
| 3 weeks | 97.3 | 95.2 | 56.7 | 95.5 | 92.1 | 53.9 |
| 4 weeks | 96.7 | 94.3 | 49.0 | 95.4 | 91.7 | 47.1 |

**Table 6**

| | Galactose | Lactose |
|---|---|---|
| -10°C | 15.8 years | 13.8 years |
| 4°C | 1.3 years | 1.2 years |

### (Example 6)

5 (W/V)% of galactose or 5 (W/V)% of lactose, or trehalose as Comparative Example was added to a 40 mM BES-NaOH buffer containing 3% of BSA, 0.5 mM of magnesium chloride, 10 µM of zinc chloride, and 400 U/L of human liver-derived ALP (Number T-73 produced by Asahi Kasei Co, Ltd.), and 2 ml of the resulting solution was injected into a vial and freeze-dried. Changes in activity were examined for 6 months in cold storage (5°C). The results thereof are shown in Table 7. The freeze-dried preparation added with trehalose decreased its activity in 4 months by 8.6%, whereas the preparation of the present invention showed no activity decrease.

**Table 7**

| Residual activity (%) | | | |
|---|---|---|---|
| | Galactose | Lactose | Trehalose |
| 1 month | 99.8 | 100.1 | 99.8 |
| 2 months | 99.9 | 99.8 | 96.4 |
| 3 months | 99.5 | 99.9 | 95.1 |
| 4 months | 99.5 | 99.6 | 91.4 |
| 6 months | 99.7 | 99.9 | |

### (Example 7)

5 (W/V)% of galactose, 5 (W/V)% of lactose, and 5 (W/V)% of fructose were each added to a 40 mM BES-NaOH buffer containing 3% of BSA, 0.5 mM of magnesium chloride, 10 µM of zinc chloride, and 400 U/L of human liver-derived ALP (Number T-73 produced by Asahi Kasei Co, Ltd.), and 2 ml of each of the obtained solutions was injected into a vial and freeze-dried. The freeze-dried product was dissolved in 2 ml of distilled water and then, left to stand at 25°C. Changes in ALP activity in the solution having the product dissolved therein are shown as the residual activity in Table 8. In each saccharide, there was no activity increase, and a stable condition continued for 48 hours.

**Table 8**

| Residual activity (%) | | | |
|---|---|---|---|
| | Lactose | Galactose | Fructose |
| 2 hours | 100.2 | 101.2 | 100.8 |
| 4 hours | 99.9 | 100.9 | 100.3 |
| 8 hours | 99.6 | 101.1 | 100.1 |
| 24 hours | 99.8 | 98.4 | 98.8 |
| 48 hours | 98.9 | 99.5 | 98.3 |

### (Example 8)

3% of dextran (molecular weight of 60000-9000: Wako Pure Chemical Industries, Ltd.), or 3% of BSA was added to a 20 mM POPSO-NaOH buffer (pH 7.5) containing 5% of galactose, 0.01 mM of zinc chloride, 0.5 mM of magnesium chloride, and 400 U/L of human liver-derived ALP, and 2 ml of each of the obtained products was injected into a vial and freeze-dried under the same freeze-drying conditions as in Example 3. Under the above respective conditions, a load test was made on each of the freeze-dried products at 37°C which was then dissolved in 2 ml of distilled water every week for up to 4 weeks at the time of measurement to measure ALP activity. Changes in the residual activity of each sample are shown in Table 9. Also in the case of dextran, a stabilizing effect could be confirmed although slightly inferior to BSA. Note that the product to which neither dextran nor BSA was added was used as a control, with the result that the product could not be freeze-dried sufficiently.

**Table 9**

| Residual activity (%) | | |
|---|---|---|
| | 3% of dextran | 3% of BSA |
| 1 week | 91.2 | 91.8 |
| 2 weeks | 84.8 | 85.2 |
| 3 weeks | 76.7 | 81.2 |
| 4 weeks | 66.2 | 77.3 |

### INDUSTRIAL APPLICABILITY

According to the present invention, there is provided, as a freeze-dried alkaline phosphatase-containing preparation for use in clinical examination, specifically, a freeze-dried preparation in which human liver alkaline phosphatase is stable, and which shows no increase in activity after reconstitution in water and can be stored for the long term.

## Claims

1. A method of stabilizing human liver alkaline phosphatase in a freeze-dried preparation, comprising freeze-drying the human liver alkaline phosphatase in the presence of a saccharide selected from the group consisting of galactose, lactose and fructose,in combination with albumin or dextran.

2. The stabilizing method according to claim 1, wherein human liver alkaline phosphatase is obtained from a recombinant cell transformed with a gene encoding, human liver alkaline phosphatase.

3. The stabilizing method according to claim 1 or claim 2, wherein albumin is used in combination with the saccharine.

## Patentansprüche

1. Verfahren zur Stabilisierung humaner alkalischer Leberphosphatase in einer gefriergetrockneten Zubereitung, umfassend das Gefriertrocknen der humanen alkalischen Leberphosphatase in der Gegenwart eines Saccharids, das ausgewählt ist aus der Gruppe, bestehend aus Galactose, Lactose und Fructose, in Verbindung mit Albumin oder Dextran.

2. Das Stabilisierungsverfahren nach Anspruch 1, wobei die humane alkalische Leberphosphatase erhalten wird aus einer rekombinanten Zelle, die mit einem Gen transformiert ist, das für die humane alkalische Leberphosphatase kodiert.

3. Das Stabilisierungsverfahren nach Anspruch 1 oder Anspruch 2, wobei Albumin in Verbindung mit dem Saccharid verwendet wird.

## Revendications

1. Procédé de stabilisation d'une phosphatase alcaline humaine de foie dans une préparation par lyophilisation, comprenant la lyophilisation de la phosphatase alcaline humaine de foie en présence d'un saccharide sélectionné dans le groupe constitué de galactose, de lactose et de fructose, en combinaison avec de l'albumine ou de la dextrane.

2. Procédé de stabilisation selon la revendication 1, dans lequel la phosphatase alcaline humaine de foie est obtenue à partir d'une cellule recombinée, transformée avec un gène codant la phosphatase alcaline humaine de foie.

3. Procédé de stabilisation selon la revendication 1 ou la revendication 2, dans lequel l'albumine est utilisée en combinaison avec le saccharide.
